Europäisches Patentamt

European Patent Office

Office européen des brevets

(19)

(11) Publication number: **0 030 475**
**B1**

(12) **EUROPEAN PATENT SPECIFICATION**

(45) Date of publication of patent specification: **14.03.84**

(21) Application number: **80304440.3**

(22) Date of filing: **09.12.80**

(51) Int. Cl.³: **C 07 D 203/08,**
**C 07 D 203/02**

(54) Process for producing solutions of aziridine-2-carboxylic acid salts.

(30) Priority: 10.12.79 JP 159250/79
12.12.79 JP 160350/79
12.12.79 JP 160351/79
25.12.79 JP 167681/79
16.01.80 JP 2566/80

(43) Date of publication of application:
**17.06.81 Bulletin 81/24**

(45) Publication of the grant of the patent:
**14.03.84 Bulletin 84/11**

(84) Designated Contracting States:
**CH DE FR GB IT LI NL**

(56) References cited:
**CHEMISCHE BERICHTE, vol. 93, no. 7, 1960,
pages 1451–1696 B. EISTERT et al.: "Die
RedoxDisproportionierung von vic.
Polycarbonylverbindungen"**

**HELVETICA CHIMICA ACTA, vol. 49, 22nd
January 1966, pages 359–369 E. KYBURZ et al.:
"Synthese und Eigenschaften von
Aziridincarbonsäureestern"**

**Chem. Ber. 93 (1960), 1632–1643**

**The file contains technical information
submitted after the application was filed and not
included in this specification**

(73) Proprietor: **MITSUI TOATSU CHEMICALS,
INCORPORATED**
**2-5 Kasumigaseki 3-chome**
**Chiyoda-Ku Tokyo 100 (JP)**

(72) Inventor: **Higuchi, Chojiro**
**4-5-13, Dai**
**Kamakura-shi Kanagawa-ken (JP)**
Inventor: **Kato, Toshio**
**600-1, Kamisakunobe Takatsu-ku**
**Kawasaki-shi Kanagawa-ken (JP)**
Inventor: **Kawashima, Nobuyuki**
**4-1-28, Hase**
**Kamakura-shi Kanagawa-ken (JP)**
Inventor: **Yamaguchi, Akihiro**
**1-1-21, Iwase**
**Kamakura-shi Kanagawa-ken (JP)**
Inventor: **Nagai, Shosuke**
**1071-2, Nakano-cho Totsuka-ku**
**Yokohama-shi Kanagawa-ken (JP)**
Inventor: **Takano, Takao**
**380-172, Watauchi**
**Fujisawa-shi Kanagawa-ken (JP)**
Inventor: **Mita, Ryuichi**
**529, Shimosakunobe Takatsu-ku**
**Kawasaki-shi Kanagawa-ken (JP)**

(74) Representative: **Armitage, Ian Michael et al,**
**MEWBURN ELLIS & CO. 2/3 Cursitor Street**
**London EC4A 1BQ (GB)**

Note: Within nine months from the publication of the mention of the grant of the European patent, any person may
give notice to the European Patent Office of opposition to the European patent granted. Notice of opposition shall
be filed in a written reasoned statement. It shall not be deemed to have been filed until the opposition fee has been
paid. (Art. 99(1) European patent convention).

Courier Press, Leamington Spa, England.

# 0 030 475

## Process for producing solutions of aziridine-2-carboxylic acid salts

This invention relates to a novel process for producing solutions of aziridine-2-carboxylic acid salts which are useful as intermediates for the production of an alpha-amino acid, an agricultural chemical, a pharmaceutical, etc.

Some methods for the production of an aziridine-2-carboxylic acid salt have been known. For example, for the production of sodium aziridine-2-carboxylate, there are known (1) a method which comprises neutralizing alpha-chloro-beta-alanine hydrochloride in water with an aqueous solution of sodium hydroxide, and while heating the neturalization product under reflux, adding a 1N aqueous solution of sodium hydroxide dropwise so that the pH of the aqueous solution is maintained at 7—7.5 [K.D. Gundermann, Chem. Ber., *93*, 1640 (1960)], and (2) a method which comprises hydrolyzing isopropyl aziridine-2-carboxylate obtained by reaction between isopropyl-alpha, beta-dibromopropionate and liquid ammonia, with sodium ethoxide in a mixed solvent consisting of ether, ethanol and water [E. Kyburz, Hev. Chim. Acta, *49*, 368 (1966)]. Lithium aziridine-2-carboxylate is known to be produced by (3) a method which comprises treating alpha-chloro-beta-alanine ethyl ester with triethanolamine in ethanol to form ethyl aziridine-2-carboxylate, and hydrolyzing the ethyl ester with lithium hydroxide in a mixed solvent consisting of ethanol and water [K. D. Gundermann, Chem. Ber., *93*, 1639 (1960)]. The method (1), however, has the defect that it has to be operated while the concentration of the starting alpha-chloro-beta-alanine hydrochloride in the reaction system is maintained at as low as about 1% by weight. The methods (2) and (3), on the other hand, have the defect that synthesis of aziridine-2-carboxylic acid ester is complex and the yield of the final product is low. Accordingly, none of these prior methods are entirely satisfactory in commercial practice.

It is desirable to provide a commercially advantageous process for producing solutions of aziridine-2-carboxylic acid salts.

The present invention provides a process which comprises treating a solution of an alpha-halogeno-beta-aminopropionitrile or its mineral acid salt in water or in a water-containing organic solvent with an alkali or alkaline earth metal hydroxide at 0°C to 100°C. According to this process, hydrolysis of the nitrile group and intramolecular dehydrochlorination (cyclization) take place, and an alkali or alkaline earth metal salt of aziridine-2-carboxylic acid can be formed almost selectively without substantially forming by-products.

We have found that it is commercially advantageous to produce solutions of aziridine-2-carboxylic acid salts by using as a starting material an alpha-halogenoacrylonitrile which can be easily produced at low cost by halogenation and dehydrohalogenation of acrylonitrile or an alpha, beta-dihalogenopropionitrile which can be produced easily at low cost on a commercial scale by halogenation of acrylonitrile. As a result, we now provide a process for producing solutions of aziridine-2-carboxylic salts characterised by treating a solution of an alpha-halogeno-beta-aminopropionitrile or its mineral acid salt in water or in a mixture of water and a water miscible organic solvent with at least two equivalents of an alkali or alkaline earth metal hydroxide in the case where the free $\alpha$-halogeno-$\beta$-amino propionitrile is used or at least three equivalents in the case where the mineral acid salt thereof is used, at a temperature of 0 to 100°C.

A solution of alpha-halogeno-beta-aminopropionitrile or its salt, obtained by reacting an alpha-halogenoacrylonitrile or an alpha, beta-dihalogenopropionitrile with ammonia in water or a mixture of water and a water-miscible organic solvent, can be used directly in the process hereof without isolating the product therefrom.

The processes of this invention have various advantages over the aforesaid known methods in that the starting materials can be produced easily at low cost, the reaction operation is simple, the process steps are markedly simplified, and the desired aziridine-2-carboxylic acid obtained in a high yield. Accordingly, they are of high utilitarian value in commercial practice.

The alpha-halogeno-beta-aminopropionitrile or its mineral acid salt used in the processes of this invention includes, for example, alpha-chloro-beta-aminopropionitrile, alpha-bromo-beta-amino-propionitrile, alpha-chloro-beta-aminopropionitrile hydrochloride and sulfate, and alpha-bromo-beta-aminopropionitrile hydrochloride and sulfate.

The alpha-halogeno-beta-aminopropionitrile or its mineral acid salt is obtained by reacting an alpha, beta-dihalogeno-propionitrile or an alpha-halogenoacrylonitrile with ammonia in water or an organic solvent. Free alpha-halogeno-beta-aminopropionitrile is obtained by distilling under reduced pressure the reaction mixture or the extract obtained by extracting the reaction product from the reaction mixture with a water-immiscible organic solvent. Action of hydrochloric acid or sulfate on the reaction mixture gives an alpha-halogeno-beta-aminopropionitrile hydrochloride or sulfate. For example, alpha-chloro-beta-aminopropionitrile hydrochloride can be isolated in a yield of more than 80% by adding dropwise alpha-chloroacrylonitrile at about 0°C to a solution of ammonia as in isopropanol, reacting them at this temperature for 2 to 4 hours, and feeding a solution of hydrogen chloride in isopropanol.

The aqueous solution or water-containing organic solvent solution containing the alpha-halogeno-beta-aminopropionitrile or its mineral acid salt used in the processes of this invention may be a reaction mixture containing an alpha-halogeno-beta-aminopropionitrile obtained by the reaction of

2

(1) an alpha, beta-dihalogenopropionitrile or (2) an alpha-halogenoacrylonitrile with ammonia. This reaction mixture is described more specifically below.

(1) First, there will be described the reaction mixture containing an alpha-halogeno-beta-amino-propionitrile obtained by reacting an alpha, beta-dihalogenopropionitrile with ammonia in water and/or an organic solvent.

The starting alpha, beta-dihalogenopropionitrile may be any of chlorine, bromine, iodine and fluorine derivatives, but alpha,beta-dichloropropionitrile and alpha,beta-dibromopropionitrile are preferred. The alpha,beta-dihalogenopropionitrile can be easily produced by halogenation of acrylonitrile.

Ammonia used in the production of the alpha-halogeno-beta-aminopropionitrile is used in the form of aqueous ammonia or a solution of ammonia gas or aqueous ammonia in an organic solvent. The reaction may also be performed while introducing ammonia gas into the reaction system. The organic solvent used in the reaction denotes an organic solvent having the ability to dissolve ammonia. Generally, it is a lower alcohol such as methanol, ethanol, n-propanol, isopropanol, n-butanol, isobutanol, tert.-butanol, methyl Cellosolve (trade mark for $\beta$-oxyethylmethylether) or Cellolsolve (trade mark for $\beta$-oxyethylether). Two or more of these organic solvents may be used in combination. Such an organic solvent may also be used as a mixture with water.

The amounts of the alpha,beta-dihalogenopropionitrile and ammonia are such that at least 2 moles, preferably at least 2.2 moles, of ammonia is used per mole of the alpha,beta-dihalogenopropionitrile. When the reaction is carried out in aqueous ammonia, ammonia is used as a 5—30% by weight solution. When the reaction is carried out in an organic solvent or a water-containing organic solvent, ammonia is used as a solution having a concentration of 2 to 25% by weight.

In the reaction of forming the alpha-halogeno-beta-aminopropionitrile, there is no particular limitation on the method and order of adding the starting material and the solvent, etc. Usually, it is preferred to add the alpha,beta-dihalogenopropionitrile gradually to water and/or an organic solvent containing water. The alpha,beta-dihalogenopropionitrile may be added after it is diluted with an organic solvent.

The reaction temperature is —40 to 30°C, preferably —20 to 20°C, and the reaction time is suitably 0.5 to 20 hours, preferably 1 to 15 hours. The reaction may be performed in an atmosphere of air. Preferably, it is carried out in an inert gas, for example in a nitrogen atmosphere or nitrogen stream, because side-reactions can be inhibited in the inert gas.

The end point of the reaction between the alpha,beta-dihalogenopropionitrile and ammonia can be rapidly and easily determined by gas chromatography, high-speed liquid chromatography, etc.

(2) The reaction mixture containing an alpha-halogeno-beta-aminopropionitrile obtained by reacting an alpha-halogenoacrylonitrile with ammonia in water and/or an organic solvent is described below specifically.

Usually, alpha-chloroacrylonitrile and alpha-bromo-acrylonitrile are frequently used as the alpha-halogenoacrylonitrile.

Ammonia is used in the form of aqueous ammonia or a solution of ammonia gas or aqueous ammonia in an organic solvent. The reaction between the alpha-halogenoacrylonitrile and ammonia may be carried out in water and/or an organic solvent. When the reaction is carried out in an organic solvent, the organic solvent used is a compound having the ability to dissolve ammonia. Suitable organic solvents are lower alcohols such as methanol, ethanol, n-propanol, isopropanol, n-butanol, isobutanol, tert.-butanol, methyl Cellosolve (trade mark for $\beta$-oxyethylmethylether) and Cellosolve (trade mark for $\beta$-oxyethylether). They are used either singly or as a mixture of two or more. These organic solvents may be used as a mixture with water.

The amounts of the alpha-halogenoacrylonitrile and ammonia are such that at least 1 mole, preferably at least 1.2 moles, of ammonia is used per mole of the alpha-halogenoacrylonitrile. When the reaction is carried out in aqueous ammonia, ammonia is used as a solution having a concentration of 5 to 30% by weight. When the reaction is carried out in an organic solvent or a water-containing organic solvent, ammonia is used as a solution having a concentration of 2 to 25% by weight.

In the reaction of the alpha-halogenoacrylonitrile with ammonia, there is no particular limitation on the method and order of adding the starting material and the solvent. Usually, it is preferred to add the alpha-halogenoacrylonitrile to water and/or an organic solvent containing ammonia. The alpha-halogenoacrylonitrile may be added after it is diluted with an organic solvent.

The reaction temperature is —40 to 0°C, preferably —20 to 20°C, and the reaction time is suitably 0.5 to 20 hours, preferably 1 to 15 hours. The reaction may be carried out in an atmosphere of air or an inert gas. The use of an inert gaseous atmosphere, for example a nitrogen atmosphere or nitrogen stream, is preferred since side-reactions can be inhibited in the inert gaseous atmosphere. The end point of the reaction can be easily and rapidly determined by gas chromatography, high-speed liquid chromatography, etc.

The alkali or alkaline earth metal hydroxide used in this invention includes hydroxides of alkali metals such as lithium, sodium, potassium and rubidium, and hydroxides of alkaline eath metals such as beryllium, magnesium, calcium, strontium and barium. Specific examples are lithium hydroxide, sodium hydroxide, potassium hydroxide, beryllium hydroxide, magnesium hydroxide, calcium hydroxide and barium hydroxide. Usually, sodium hydroxide, potassium hydroxide and calcium hydroxide are preferred.

In the processes of this invention, the amount of the alkali or alkaline eath metal hydroxide is at least 2 equivalents based on the starting material when it is a free alpha-halogeno-beta-aminopropionitrile, and at least 3 equivalents based on the starting material when it is a mineral acid salt of an alpha-halogeno-beta-aminopropionitrile. There is no particular upper limit to the amount of the hydroxide. But it is not necessary to use it in large excess, and usually up to 5 equivalents are sufficient.

When the reaction mixture from the alpha,beta-dihalogenopropionitrile or alpha-halogenoacrylonitrile is subsequently treated with alkalies, the amount of the alkali or alkaline earth metal used is at least 2 equivalents, preferably at least 2.2 equivalents, based on the alpha,beta-dihalogenopropionitrile or alpha-halogenoacrylonitrile. There is no particular limitation to the upper limit of the amount of the alkali or alkaline earth metal. But is is not necessary to use it in large excess, and usually, up to 5 equivalents are sufficient.

The processes of this invention are carried out in water or in a water-containing organic solvent. In other words, the reaction is carried out in an aqueous solution, or in a mixture of water and a water-miscible organic solvent.

Examples of the water-miscible organic solvent are methanol, ethanol, n-propanol, isopropanol, tert. butanol, Cellosolve (trade mark for $\beta$-oxyethylether), methyl Cellosolve (trade mark for $\beta$-oxyethylmethylether), acetone, dioxane, tetrahydrofuran, N,N-dimethyl formamide, N,N-diethyl formamide and dimethyl sulfoxide. When water and the organic solvent are used in combination, the ratio of water to the organic solvent may be chosen as desired. The amount of the solvent used is suitably 3 to 200 times, preferably 5 to 100 times, the amount of the starting alpha-halogeno-beta-aminopropionitrile or its mineral acid salt.

In performing the processes of this invention, there is no limitation on the method and order of adding the starting material, the alkali and the reaction solvent. Usually, a predetermined amount of the alkali or alkaline earth metal hydroxide in the form of an aqueous solution or suspension is added to a solution of the alpha-halogeno-beta-aminopropionitrile or its mineral acid salt in water and/or a water-miscible organic solvent in a desired concentration. Or conversely, the solution containing the alpha-halogeno-beta-aminopropionitrile may be added to the aqueous solution or suspension of the alkali or alkaline earth metal hydroxide. When the reaction mixture containing an alpha-halogeno-beta-aminopropionitrile derived from an alpha, beta-dihalogenopropionitrile or an alpha-halogenoacrylonitrile is used, it is possible, if desired, to treat a solution of the reaction mixture in water and/or the organic solvent with the hydroxide in the same manner as above.

The alkali or alkaline earth metal hydroxide may be added as a solid.

When by-product ammonium chloride precipitates from the reaction mixture containing an alpha-halogeno-beta-aminopropionitrile, the reaction mixture is submitted to hydrolysis after separating tha ammonium chloride from it. When the reaction of forming the alpha-halogeno-beta-aminopropionitrile is carried out in an organic solvent, water should be added for the subsequent hydrolysis and cyclization reaction. The amount of water to be added is not particularly limited, and the ratio between the organic solvent and water can be chosen as desired.

The reaction temperature is 0 to 100°C, preferably 20 to 80°C. The reaction is carried out usually at atmospheric pressure, but if desired, it may be performed under reduced or elevated pressure. The reaction time varies depending upon the various reaction conditions, but is usually 0.5 to 50 hours, particularly 2.0 to 30 hours. The end point of the reaction can be easily and rapidly determined by thin-layer chromatography.

In the processes of this invention, aziridine-2-carboxylic acid is formed as an alkali metal salt or an alkaline earth metal salt corresponding to the alkali used in the reaction. If desired, the organic solvent may be removed under reduced pressure from the product to obtain the product in the form of an aqueous solution.

The following Examples illustrate the processes of this invention more specifically.

Lithium aziridine-2-carboxylate as a standard sample used in the Examples was synthesized in the following manner (after K.D. Gundermann, Chem. Ber. *93* (1960), 1638—1639). Analysis by high-speed liquid chromatography was performed under the following conditions:

(a) Production of lithium aziridine-2-carboxylate

10 g of ethyl alpha-chloro-beta-aminopropionate hydrochloride was dissolved in 100 ml of dehydrated ethanol, and 20 g of triethanolamine was added. With stirring, they were reacted at 60 to 70°C for 5 hours. The precipitated triethanolamine hydrochloride was separated by filtration, and washed with a small amount of ethanol. The filtrate and the washing were combined, and with cooling, 55 ml of a 1N aqueous solution of lithium hydroxide was gradually added. The mixture was allowed to stand for 24 hours in a cold dark place. The reaction mixture was then concentrated to dryness under reduced pressure. Then, 30 ml of benzene was added to the residue (syrupy material), and water was completely removed by azeotropic distillation. Then, 50 ml of hot ethanol was added, and the mixture was cooled to form a precipitate. 100 ml of ether was added to precipitate the crystals fully. The precipitate was separated by filtration, and washed with ether to afford 1.0 g of lithium aziridine-2-carboxylate.

Melting point: 260—268°C (decomp.)

Elemental analysis (%) for $C_3H_4NO_2Li$:

|  | C | H | N | Li |
|---|---|---|---|---|
| Found (%): | 37.86 | 4.23 | 14.71 | 7.40 |
| Calculated (%): | 38.74 | 4.33 | 15.06 | 7.46 |

The purity of the resulting lithium aziridine-2-carboxylate, determined by proton NMR spectroscopy using dioxane as an internal standard (measuring solvent: $D_2O$, measuring temperature: room temperature), was 97%.

(b) Conditions for analysis by high-speed liquid chromatography
Column: Shodex OH Pak B-804 (a product of Showa Denko Co., Ltd.)
Detector: Differential refractometer RI-2 (a product of Nippon Bunseki Kogyo Co., Ltd.)
Mobile phase: Aqueous solution of $H_3PO_4$ ($1 \times 10^{-3}$ mole/liter)
Flow rate: 1.3 ml/min.
Measuring temperature: room temperature

Under these conditions, the retention time of aziridine-2-carboxylic acid was 16.5 t 16.8 minutes.

Example 1

28.2 g of alpha-chloro-beta-aminopropionitrile hydrochloride was dissolved in 80 g of water. Then, with stirring, an aqueous solution of 25.6 g of sodium hydroxide in 84 g of water was added dropwise, and they were reacted at room temperature for 24 hours. The reaction mixture was analyzed by high-speed liquid chromatography using lithium aziridine-2-carboxylate as a standard sample. The yield of sodium aziridine-2-carboxylate formed was 95.5% based on the alpha-chloro-beta-aminopropionitrile hydrochloride.

When the resulting reaction mixture was analyzed by proton NMR spectroscopy, a signal attributed to the methylene proton of aziridine-2-carboxylic acid was detected at $\delta = 1.79$ ppm (q., 2H), and a signal attributed to the methine protone, at $\delta = 2.35$ ppm (q., 1H). This signal pattern was identical with that of lithium aziridine-2-carboxylate synthesized as a standard sample.

Example 2

14.1 g of alpha-chloro-beta-aminopropionitrile hydrochloride was dissolved in 160 g of water. With stirring, an aqueous solution of 22.4 g of potassium hydroxide in 90 g of water was added dropwise. Then, the reaction mixture was heated to 60°C, and reacted at 60 to 65°C for 4 hours. The reaction mixture was analyzed by high-speed liquid chromatography in the same was as Example 1. The yield of potassium aziridine-2-carboxylate formed was 92.3%.

Example 3

Example 2 was repeated except that 10.5 g of free alpha-chloro-beta-aminopropionitrile was used instead of the alpha-chloro-beta-aminopropionitrile hydrochloride in Example 2, and 13 g of lithium hydroxide was used instead of potassium hydroxide. The yield of lithium aziridine-2-carboxylate formed was 90.8%.

Example 4

Example 2 was repeated except that 15.3 g of alpha-chloro-beta-aminopropionitrile sulfate was used instead of the alpha-chloro-beta-aminopropionitrile hydrochloride in Example 2. The yield of lithium aziridine-2-carboxylate formed was 94.2%.

Example 5

14.1 g of alpha-chloro-beta-aminopropionitrile hydrochloride was dissolved in 160 g of water, and with stirring, 12 g of calcium hydroxide was gradually added. Then, the reaction mixture was heated to 60°C, and reacted at 60 to 65°C for 8 hours. After the reaction, the excess of calcium hydroxide was removed by filtration. The residue was analyzed by high-speed liquid chromatography in the same way as in Example 1. The yield of calcium aziridine-2-carboxylate formed was 92.5%.

Example 6

14.1 g of alpha-chloro-beta-aminopropionitrile hydrochloride was dissolved in 150 g of methanol, and with stirring an aqueous solution of 12.8 g of sodium hydroxide in 75 g of water was added

5

dropwise. Then, the reaction mixture was heated to 60°C, and reacted at this temperature for 10 hours. Thereafter, the methanol in the reaction mixture was distilled off under reduced pressure, and the residue was analyzed by high-speed liquid chromatography. The yield of sodium aziridine-2-carboxylate formed was 89.2%.

Example 7

Example 6 was repeated except that 150 g of isopropanol was used instead of the methanol. The yield of sodium aziridine-2-carboxylate formed wad 93.5%.

Example 8

60.8 g of conc. aqueous ammonia (concentration 28% by weight) was maintained at 0°C, and with stirring in gentle stream of nitrogen, 24.8 g of alpha,beta-dichloropropionitrile was added dropwise over the course of about 2 hours. The reaction was performed further for 3 hours at 0 to 5°C. Then, an aqueous solution of 25.6 g of sodium hydroxide in 150 g of water was added dropwise over the course of about 30 minutes, and the reaction was performed further at room temperature for 30 hours. The reaction mixture was analyzed by high-speed liquid chromatography. The yield of sodium aziridine-2-carboxylate formed was 80.1% based on the alpha,beta-dichloropionitrile. The retention time was 16.5 minutes.

In a proton NMR spectrum of the reaction mixture (measuring temperature: room temperature), signals were observed only at $\delta$=1.79 ppm (q., 2H) and $\delta$=2.35 ppm (q., 1H). These signals were identical with those of the lithium aziridine-2-carboxylate as a standard sample in aqueous solution.

Example 9

225 g of an isopropanol solution having dissolved therein ammonia gas in a concentration of 6.8% by weight was maintained at 0°C, and in a gentle stream of nitrogen, 24.8 g of alpha,beta-dichloropropionitrile was added dropwise over the course of about 2 hours with stirring. Then, the reaction was performed at 0 to 5°C for 3 hours to form alpha-chloro-beta-aminopropionitrile. Then, while the reaction mixture was externally cooled, an aqueous solution of 25.6 g of sodium hydroxide in 230 g of water was gradually added dropwise. The solution was then heated to 50°C, and reacted at 50 to 55°C for 7 hours. Isopropanol was distilled off from the reaction mixture under reduced pressure, and the residue was analyzed by high-speed liquid chromatography in the same way as in Example 8. The yield of sodium aziridine-2-carboxylate formed was 90.8% based on the alpha,beta-dichloro-propionitrile.

Examples 10 to 13

Example 9 was repeated except that each of the alkalies shown in Table 1 was used instead of sodium hydroxide. An aqueous solution of an alkali or alkaline earth metal aziridine-2-carboxylate corresponding to the used alkali was obtained. The yield of the final product formed was as shown in Table 1.

TABLE 1

| | Alkali used | | Yield of the aziridine-2-carboxylate based on alpha, beta-dichloropropionitrile |
|---|---|---|---|
| Example | Type | Amount (g) | (%) |
| 10 | Lithium hydroxide | 25.2 | 91.5 |
| 11 | Potassium hydroxide | 35.9 | 89.7 |
| 12 | Calcium hydroxide | 23.7 | 89.4 |
| 13 | Magnesium hydroxide | 18.7 | 88.6 |

Examples 14 to 16

Example 9 was repeated except that, each of the solvents shown in Table 2 was used instead of isopropanol. The yield of the resulting sodium aziridine-2-carboxylate is shown in Table 2.

## TABLE 2

| Example | Solvent | Yield of sodium aziridine-2-carboxylate based on alpha, beta-dichloropropionitrile (%) |
|---|---|---|
| 14 | Methanol | 86.3 |
| 15 | Ethanol | 90.4 |
| 16 | Methyl Cellosolve (trade mark for $\beta$-oxyethyl-methylether) | 87.6 |

### Example 17

Example 9 was repeated except that the reaction between alpha,beta-dichloropropionitrile and ammonia was carried out at 15 to 20°C. An aqueous solution of sodium aziridine-2-carboxylate was obtained in a yield of 91.3 mole % based on the alpha,beta-dichloropropionitrile.

### Example 18

A solution obtained by dissolving 17 g of ammonia gas in 200 g of hydrous ethanol containing 20% by weight of water was cooled to 0°C, and in a gentle stream of nitrogen, 24.8 g of alpha,beta-dichloropropionitrile was added dropwise over the course of about 2 hours with stirring. The reaction was performed further at 0 to 5°C for 4 hours to form alpha-chloro-beta-aminopropionitrile. Then, the reaction mixture was added dropwise to an aqueous solution of 25.6 g of sodium hydroxide in 200 g of water with external cooling. The solution was then heated to 60°C, and reacted at 60 to 65°C for 5 hours. Ethanol was distilled off from the reaction mixture under reduced pressure, and the residue was analyzed by high-speed liquid chromatography in the same way as in Example 1. The yield of sodium aziridine-2-carboxylate formed was 86.7% based on the alpha,beta-dichloropropionitrile.

### Example 19

60.8 g of conc. aqueous ammonia (concentration 28%) was maintained at 0°C, and with stirring in a gentle stream of nitrogen, 17.5 g of alpha-acrylonitrile was added dropwise over the course of about 2 hours. Then, the reaction was performed at 0 to 50°C for 4 hours. An aqueous solution of 17.6 g of sodium hydroxide in 120 g of water was added dropwise over the course of about 30 minutes, and the reaction was performed further at room temperature for 30 hours. The reaction mixture was analyzed by high speed liquid chromatography. The yield of sodium aziridine-2-carboxylate formed was 78.6% based on the alpha-chloroacrylonitrile.

In a proton NMR spectrum (measuring temperature: room temperature) of the reaction mixture, signals were observed only at $\delta=1.79$ ppm (q., 2H) and $\delta=2.35$ ppm q., 1H). These signals were identical with those of lithium aziridine-2-carboxylate as a standard sample in aqueous solution.

### Example 20

200 g of an isopropanol solution having dissolved therein ammonia gas in a concentration of 6.8% by weight was maintained at 0°C, and in a gentle stream of nitrogen, 17.5 g of alpha-chloroacrylonitrile was gradually added dropwise over the course of about 1.5 hours with stirring. Thereafter, the reaction was performed at 0 to 5°C for 2 hours to form alpha-chloro-beta-aminopropionitrile. Then, while the reaction mixture was cooled externally, a solution of 17.6 g of sodium hydroxide in 200 g of water was gradually added dropwise. The solution was heated to 50°C, and reacted at 50 to 60°C for 8 hours. Isopropanol was distilled off from the reaction mixture under reduced pressure, and the residue was analyzed by high-speed liquid chromatography. The yield of sodium aziridine-2-carboxylate formed was 88% based on the alpha-chloroacrylonitrile.

### Examples 21 to 23

Example 20 was repeated except that each of the alkalies shown in Table 3 was used instead of sodium hydroxide. An aqueous solution of the corresponding alkali or alkaline earth metal salt of aziridine-2-carboxylic acid was obtained in the yields shown in Table 3.

7

TABLE 3

| Example | Alkali used | | Yield of the aziridine-2-carboxylate salt based on alpha-chloro-acrylonitrile (%) |
|---|---|---|---|
| | Type | Amount (g) | |
| 21 | Lithium hydroxide | 18.5 | 87.5 |
| 22 | Potassium hydroxide | 25.0 | 89.6 |
| 23 | Calcium hydroxide | 16.3 | 90 1 |

Example 24

Example 20 was repeated except that methanol was used instead of isopropanol. An aqueous solution of sodium aziridine-2-carboxylate was obtained in a yield of 85.1%.

Example 25

Example 20 was repeated except that the reaction between alpha-chloroacrylonitrile and ammonia was carried out at 15 to 20°C. An aqueous solution of sodium aziridine-2-carboxylate was obtained in a yield of 90.4%.

**Claims**

1. A process for producing solutions of aziridine-2-carboxylic salts characterized by treating a solution of an alpha-halogeno-beta-aminopropionitrile or its mineral acid salt in water or in a mixture of water and a water miscible organic solvent with at least two equivalents of an alkali or alkaline earth metal hydroxide in the case where the free-$\alpha$-halogeno-$\beta$-aminopropionitrile is used or at least three equivalents in the case where the mineral acid salt thereof is used, at a temperature of 0 to 100°C.

2. The process of claim 1 wherein the alpha-halogeno-beta-aminopropionitrile or its mineral acid salt is alpha-chloro-beta-aminopropionitrile or alpha-bromo-beta-amino-propionitrile or the hydrochloride or sulfate thereof.

3. The process of claim 1 or 2 wherein the solution of the alpha-halogeno-beta-aminopropionitrile or its mineral acid salt is a reaction mixture obtained by reacting an alpha-halogenoacrylonitrile with at least one mole of ammonia per mole of the alpha-halogenoacrylonitrile in water or in a mixture of water and a water-miscible organic solvent at a temperature of −40 to 30°C.

4. The process of claim 1 or 2 wherein the solution of the alpha-halogeno-beta-aminopropionitrile or its mineral acid salt is a reaction mixture obtained by reacting an alpha,beta-dihalogeno-propionitrile with at least two moles of ammonia per mole of the alpha,beta-propionitrile in water or in a mixture of water and a water-miscible organic solvent at a temperature of −40 to 30°C.

5. The process of any one of the preceding claims wherein the water-miscible organic solvent is methanol, ethanol, n-propanol, isopropanol, tert-butanol, $\beta$-oxyethylether, $\beta$-oxyethylmethylether, acetone, dioxane, tetrahydrofuran, N,N-diethyl formamide, N,N-dimethyl formamide or dimethyl sulfoxide.

6. The process of any one of the preceding claims wherein the alkali or alkaline earth metal hydroxide is sodium hydroxide, potassium hydroxide or calcium hydroxide.

**Revendications**

1. Procédé de production de solutions de sels aziridine-2-carboxyliques, caractérisé par le traitement d'une solution d'un alpha-halogéno-béta-aminopropionitrile ou son sel d'acide minéral dans l'eau ou dans un mélange d'eau et d'un solvant organique miscible dans l'eau avec au moins deux équivalents d'un hdyroxyde d'un métal alcalin ou alcalino-terreux dans le cas où l'on utilise l'$\alpha$-halogéno-$\beta$-aminopropionitrile libre ou au moins trois équivalents dans le cas où l'on utilise son sel d'acide minéral, à une température de 0 à 100°C.

2. Procédé selon la revendication 1, où l'alpha-halogéno-béta-aminopropionitrile ou son sel d'acide minéral est l'alpha-chloro-béta-aminopropionitrile ou l'alpha-bromo-béta-amino-propionitrile ou son chlorhydrate ou son sulfate.

3. Procédé selon la revendication 1 ou 2, où la solution de l'alpha-halogéno-béta-aminopropionitrile ou son sel d'acide minéral est un mélange réactionnel obtenu par réaction d'un alpha-halogéno-acrylonitrile avec au moins une mole d'ammoniac par mole de l'alpha-halogéno-acrylonitrile dans l'eau ou dans un mélange d'eau et d'un solvant organique miscible dans l'eau à une température de a40 à +30°C.

4. Procédé selon la revendication 1 ou 2, où la solution de l'alpha-halogéno-béta-aminopropionitrile ou son sel d'acide minéral est un mélange réactionnel obtenu par réaction d'un alpha,béta-di- halogénopropionitrile avec au moins deux moles d'ammoniac par mole de l'alpha, béta-propionitrile dans l'eau ou dans un mélange d'eau et d'un solvant organique miscible dans l'eau à une température de −40 à 30°C.

5. Procédé selon l'une quelconque des revendications précédentes où le solvant organique miscible dans l'eau est du méthanol, de l'éthanol, du n-propanol, de l'isopropanol, du tert-butanol, du $\beta$-oxyéthyléther, du $\beta$-oxyéthylméthyléther, de l'acétone, du dioxane, du tétrahydrofurane, du N,N-diéthyl formamide, du N,N-diméthyl formamide ou du diméthyl sulfoxyde.

6. Procédé selon l'une quelconque des revendications précédentes, où l'hydroxyde d'un métal alcalin ou d'un métal alcalino-terreux est l'hydroxyde de sodium, l'hydroxyde de potassium ou l'hydroxyde de calcium.

**Patentansprüche**

1. Verfahren zur Herstellung von Lösungen von Aziridin-2-carbonsäuresalzen, gekennzeichnet durch behandeln einer Lösung eines $\alpha$-Halogen-$\beta$-aminopropionitrils oder dessen Mineralsäuresalzes in Wasser oder in einem Gemisch aus Wasser und einem mit Wasser mischbaren organischen Lösungsmittel mit wenigstens zwei Äquivalenten eines Alkali- oder Erdalkalimetallhydroxids in dem Falle, wo das freie $\alpha$-Halogen-$\beta$-aminopropionitril verwendet wird, oder wenigstens drei Äquivalenten in dem Falle, wo dessen Mineralsäuresalz verwendet wird, bei einer Temperatur von 0 bis 100°C.

2. Verfahren nach Anspruch 1, wobei das $\alpha$-Halogen-$\beta$-Aminopropionitril oder dessen Mineralsäuresalz $\alpha$-Chlor-$\beta$-aminopropionitril oder $\alpha$-Brom-$\beta$-amino-propionitril oder dessen Hydrochlorid oder Sulfat ist.

3. Verfahren nach Anspruch 1 oder 2, wobei die Lösung des $\alpha$-Halogen-$\beta$-aminopropionitrils oder dessen Mineralsäuresalzes ein Reaktionsgemisch ist, erhalten durch Umsetzen eines $\alpha$-Halogenacrylnitrils mit wenigstens einem Mol Ammoniak pro Mol des $\alpha$-Halogenacrylnitrils in Wasser oder in einem Gemisch aus Wasser und einem mit Wasser mischbaren organischen Lösungsmittel bei einer Temperatur von −40 bis 30°C.

4. Verfahren nach Anspruch 1 oder 2, wobei die Lösung des $\alpha$-Halogen-$\beta$-aminopropionitrils oder dessen Mineralsäuresalzes ein Reaktionsgemisch ist, erhalten durch Umsetzen eines $\alpha,\beta$-Dihalogenpropionitrils mit wenigstens 2 Mol Ammoniak pro Mol des $\alpha,\beta$-Propionitrils in Wasser oder in einem Gemisch aus Wasser und einem mit Wasser mischbaren organischen Lösungsmittel bei einer Temperatur von −40 bis 30°C.

5. Verfahren nach irgend einem der vorhergehenden Ansprüche, wobei das mit Wasser mischbare organische Lösungsmittel Methanol, Ethanol, n-Propanol, Isopropanol, tert.-Butanol, $\beta$-Oxyethylether, $\beta$-Oxyethylmethylether, Aceton, Dioxan, Tetrahydrofuran, N,N-Diethylformamid, N,N-Dimethylformamid oder Dimethylsulfoxid ist.

6. Verfahren nach irgend einem der vorhergehenden Ansprüche, wobei das Alkali- oder Erdalkalimetall-hydroxid Natriumhydroxid, Kaliumhydroxid oder Calciumhydroxid ist.